(19) Europäisches Patentamt
European Patent Office
Office européen des brevets

(11) Publication number: **0 546 099 B1**

(12) # EUROPEAN PATENT SPECIFICATION

(45) Date of publication of patent specification: **12.10.94**

(51) Int. Cl.5: **A61K 37/66**, C07K 13/00

(21) Application number: **91917729.5**

(22) Date of filing: **29.08.91**

(86) International application number: **PCT/US91/05982**

(87) International publication number: **WO 92/04377 (19.03.92 92/07)**

(54) **Use of human Interferon gamma 4-134.**

(30) Priority: **31.08.90 US 576245**

(43) Date of publication of application: **16.06.93 Bulletin 93/24**

(45) Publication of the grant of the patent: **12.10.94 Bulletin 94/41**

(84) Designated Contracting States: **AT BE CH DE DK ES FR GB GR IT LI LU NL SE**

(56) References cited:
**EP-A- 0 146 354**
**EP-A- 0 166 993**
**EP-A- 0 241 725**
**EP-A- 0 256 424**
**EP-A- 0 273 373**
**EP-A- 0 306 870**
**WO-A-90/02570**

(73) Proprietor: **SCHERING CORPORATION**
**2000 Galloping Hill Road**
**Kenilworth New Jersey 07033 (US)**

(72) Inventor: **NARULA, Satwant, K.**
**26 Natalie Drive**
**West Caldwell, NJ 07006 (US)**
Inventor: **LUNDELL, Daniel, J.**
**21 Sleepy Hollow Drive**
**Flemington, NJ 08822 (US)**

(74) Representative: **Ritter, Stephen David et al**
**Mathys & Squire**
**10 Fleet Street**
**London EC4Y 1AY (GB)**

**EP 0 546 099 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

The present invention relates to a particular human gamma interferon, IFNγ 4-134, and functional equivalents thereof and methods and compositions using these materials.

Human gamma interferon (usually referred to herein as Hu IFNγ) has antiviral, antiproliferative, and immunoregulatory activities (Trinchieri *et al.*, Immunol. Today, **6**, 131-136 (1985)). The cDNA for human IFNγ was cloned and expressed in *E. coli* to yield large quantities of the protein Hu IFNγ (Gray *et al.*, Nature, **295**, 503-508 (1982)). Several earlier studies have indicated that the carboxyl terminus of IFNγ may be an important structural and functional feature of this molecule. (Favre *et al.*, Molecular Immunology, 26:17-25 (1988); Burton *et al.*, (1985) H. Kirchner and H. Schellekens (eds.) The Biology of the Interferon System 1984, Elsevier Science Publishers, pp. 403-409; Honda *et al.*, J. Int. Res., **5**, 145-154 (1987); Rinderknecht *et al.*, J. Biol. Chem., **259**, 6790-6797 (1984); Russell *et al.*, J. Int. Res., **8**, 433-439 (1988); Dobeli *et al.*, J. of Biotechnology, **7**, 199-216 (1988)). Most of these studies were carried out with proteolytic degradation products.

Hu IFNγ is a polypeptide having a sequence of 146 amino acid residues as defined by SEQ ID NO: 1 herein (immediately before the Claims). The numbers used therein refer to the amino acid sequence in wild-type IFNγ; these numbers are also used in amino acid sequences whenever referred to in the text of this specification.

A considerable number of researchers have examined carboxyl-terminal variants of Hu IFNγ. This work was stimulated by the observation that in natural Hu IFNγ the carboxyl terminus is heterogeneous. Rinderknecht *et al.*, *supra,* showed that the longest natural Hu IFNγ species ended at amino acid 137, but others ended at amino acids 135, 133, 132, 131, and 130. Pan *et al.*, Eur. J. Biochem., **166**, 145-149 (1987), showed a slightly different heterogeneity at the carboxyl terminus. Dobeli *et al.*, *supra,* have made the most complete analysis of genetically engineered forms of Hu IFNγ that showed deletions at the carboxyl terminus, generally referred to as "deletion mutants" below. They showed that a large increase in biological activity, in particular antiviral and antiproliferative activities and macrophage activation, occurred when 9 or 10 amino acids were removed from the carboxyl terminus. When 14 or 18 residues were removed, this activity dropped to less than that of the full-length molecule.

From EP-A-0 146 354 a truncated human interferon-γ having an amino acid sequence defined by residues 4 to 134 of SEQ ID NO: 1 is known to have antiviral and antiproliferative properties.

According to the present invention there is provided the use of Hu IFN-γ polypeptide, whose sequence consists of residues 4-134 of SEQ ID NO: 1, or of said sequence preceded by Met, for the manufacture of a medicament for (i) increasing the antibody response in conjunction with a vaccine, (ii) increasing the susceptibility of tumor cells to cytotoxic cells and (iii) increasing the expression of surface antigens by tumor cells.

We have now surprisingly discovered that a polypeptide having an amino acid sequence corresponding to the 4th through the 134th amino acids of full length Hu $IFN_{\gamma}$ (1-146) possesses very advantageous properties. In particular, we have found that the protein of the invention, designated $IFN_{\gamma}$ 4-134, and functional equivalents thereof as described below, possess essentially none of the antiviral activity of $IFN_{\gamma}$4-137, but retain essentially all of the HLA-DR-induction activity of $IFN_{\gamma}$ 4-137. Thus, $IFN_{\gamma}$ 4-134 and its functional equivalents will provide the antigen induction of $IFN_{\gamma}$ 4-137 and other similar Hu $IFN_{\gamma}$s without the inflammatory side effects normally associated with antiviral activity of $IFN_{\gamma}$. The $IFN_{\gamma}$ 4-134 and its functional equivalents can be advantageously employed as an adjunct to a vaccine to increase antibody response. Also, because IFNγ 4-134 and functional equivalents thereof increase surface antigen expression in tumor cells, such cells become more susceptible to the actions of cytotoxic cells; and IFNγ 4-134 and functional equivalents thereof may therefore be used as anti-tumor agents.

The invention also provides recombinant vectors comprising a DNA sequence coding for a truncated recombinant human interferon-γ having an amino acid sequence defined by the sequence of residues numbered 4 to 134 of SEQ ID NO: 1 herein, or a functional equivalent thereof, which recombinant vectors are capable of directing expression of the DNA sequence in a compatible host organism (*i.e.*, the DNA sequence in operative association with an expression control sequence therefor). This invention still further provides microorganisms containing such recombinant vectors (*i.e.*, vectors capable of expressing the IFNγ 4-134 polypeptide or functional equivalent thereof in the microorganism).

The invention also provides pharmaceutical compositions comprising IFNγ 4-134 or a functional equivalent thereof in combination with a pharmaceutically acceptable carrier or excipient. Such compositions may also comprise an immuno-potentiating amount of a human lymphokine, especially a recombinant human lymphokine, selected from GM-CSF, G-CSF, M-CSF, IL-3, IL-4, IL-5, IL-6 and IL-7. The following references, all of which are hereby incorporated herein by reference, may be cited as general references to these lymphokines: Lee *et al.*, "Isolation of cDNA for a human granulocate-macrophage colony-stimulating factor by functional expression in mammalian cells", Proc. Natl. Acad. Sci. USA, 82:4360-4364 (1985); Souza *et al.*, "Recombinant Human Granulocyte Colony-Stimulating Factor: Effects on Normal and Leukemic Myeloid Cells", Science, 232:61-65 (1986); Yang *et al.*, "Human IL-3 (Multi-CSF): Identification by Expression Cloning of a Novel Hematopoietic Growth Factor Related to Murine IL-3", Cell, 47:3-10 (1986); Yokota *et al.*, "Isolation and characterization of a human interleukin cDNA clone, homologous to mouse B-cell stimulatory factor 1, that expresses B-cell- and T-cell-stimulating activities", Proc. Natl. Acad. Sci. USA, 83:5894-5898 (1986); Takatsu *et al.*, "Interleukin 5, a T-cell-derived B-cell differentiation factor also induces cytotoxic T lymphocytes", Proc. Natl. Acad. Sci. USA, 84:4234-4238 (1987); Chiu *et al.*, Proc. Natl. Acad. Sci. USA, 85:7099-7103 (1988); and Chazen *et al.*, Proc. Natl. Acad. Sci. USA, 86:5923-5927 (1989); and also the following patents: EP A 0 202 300, PCT/EP 85/00326 (published as WO 86/00639), and PCT/US 86/02464 (published as WO 87/02990).

Other embodiments of the invention provide a method for increasing the antibody response in conjunction with a vaccine, a method for increasing the susceptibility of tumor cells to cytotoxic cells, and a method for increasing the expression of surface antigens by tumor cells, all by administering IFNγ 4-134 or a functional equivalent thereof. The invention thus further provides a pharmaceutical composition comprising vaccines together with IFNγ 4-134 or a functional equivalent thereof.

## BRIEF DESCRIPTION OF THE FIGURES

Figure 1 is a schematic representation showing the construction of PIN5GIF54, a plasmid carrying the full-length IFNγ (1-146).

Figure 2 is a schematic representation showing the construction of the plasmids PIN5TGIF54 and PIN5T4.

Figure 3 is a schematic representation showing the construction of pGIF4-125 and pGIF4-137 using the carboxy-terminal deletion mutants derived by the primer repair method.

Figure 4 is a schematic representation showing the construction of plasmid PJF13-3-6.

## DETAILED DESCRIPTION OF THE INVENTION

All references cited herein are hereby incorporated herein by reference.

Because of the degeneracy of the genetic code, it will be understood that there are many potential nucleotide sequences that could code for the amino acid sequence of IFNγ 4-134 and functional equivalents thereof. It should also be understood that the nucleotide sequences of the DNA sequences of the invention inserted into vectors may include nucleotides (*e.g.*, introns) which are not part of the actual structural genes, as long as the recombinant vectors containing such sequences are capable of directing the production in an appropriate host organism of IFNγ 4-134 or a functional equivalent thereof.

Moreover, amino acid substitutions in proteins which do not essentially alter biological and immunological activities have been known to occur and have been described, *e.g.*, by Neurath *et al.* in "The Proteins", Academic Press, New York (1979), in particular in Fig. 6 at page 14. The most frequently observed amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and vice versa. These substitutions are intended as possible substitutions in the IFNγ 4-134 amino acid sequence and thus provide the functional equivalents of IFNγ 4-134. Also, another IFNγ 4-134 functional equivalent of the invention may include an initial Met amino acid before the number 4 amino acid of the 4-134 sequence as shown above. Other IFNγ 4-134 functional equivalents of the invention may lack the Gln and/or the Asp in the 4- and 5-positions respectively of the 4-134 sequence as shown above. Thus, by these functional equivalents of IFNγ 4-134, we mean polypeptides of 129 amino acids (*e.g.*, IFNγ6-134), 130 amino acids (*e.g.* IFNγ 5-134 or Met-6-134), 131 amino acids (*e.g.* IFNγ 4-134 or Met-5-134) or 132 amino acids (*e.g.* IFNγ Met-4-134) which have less than 4%, preferably less than 3% and most preferably less than 1%, of the antiviral activity of IFNγ 4-137 in the antiviral assay described below and more than 50%, preferably more than 70% and most preferably more than 85%, of the HLA-DR induction activity of IFNγ 4-137 in the assay entitled Cytofluorometric Determination of Class II MHC (HLA-DR) as described below and which have one or more of the amino acid sequence variations in the IFNγ 4-134 sequence described in this paragraph. The preferred form of the invention is IFNγ 4-134 itself rather than a functional equivalent thereof.

Preferably, the recombinant IFNγ 4-134 or functional equivalent thereof is substantially freed from all other proteins. By "substantially free" we mean that the IFNγ 4-134 or functional equivalent thereof contains less than 1% (w/w), and preferably less than 0.5% (w/w), of contaminating proteins, especially proteins originating from the genetically engineered host cell (*e.g.*, *E. coli*), and other forms of IFNγ.

The IFNγ 4-134 and functional equivalents thereof can be prepared by techniques conventional in the art. Typically, the gene or cDNA for the appropriate IFNγ 4-134 or functional equivalent thereof is first prepared and isolated. A number of different methods can be practiced to obtain these DNA materials. Preferably, the primer repair method as described by Gray *et al.* (Proc. Nat. Acad. Sci. USA, 80, 5842-5846 (1983)) is employed, as schematically shown in the following Scheme. In this method, an oligomer complementary to the appropriate site on the gene is synthesized and used to hybridize with denaturated DNA fragments carrying the gene of interest, in this case that encoding IFNγ 4-146. This technique can be used to obtain the various necessary deletion mutants of IFNγ 4-146.

In this method, the oligomers given in SEQ ID NOS: 2 and 3 can be used for primer repair and thus for defining the carboxy-terminal deletion mutants: they introduce a STOP triplet after position 125 or 137 respectively of Hu IFNγ. The initial C (cytosine) reconstructs a filled-in *Sal*I end to the full *Sal*I recognition sequence 5'-GTCGAC-3'. The first triplet CTA of each SEQ ID NO: 2 or 3 represents the STOP triplet (corresponding to TAG on the coding strand). The next triplet, CGG or CAT respectively, encodes the last amino acid for the polypeptide, viz 125 Pro or 137 Met respectively.

The following Scheme I shows how the primer repair method of Gray *et al.* (Proc. Natl. Acad. Sci. USA, cited above) can be used to provide an oligonucleotide encoding the desired fraction of IFNγ. The double-stranded fragment is then ligated into the desired plasmid, *e.g.* as shown in Figure 3, where X indicates the STOP triplet, and the underlined slashes (/////) indicate the part of the single-stranded DNA that is removed by Klenow fragment (3'-5' exonuclease and 5'-3' polymerase):

## SCHEME I

**Preliminary Step:**

Isolate the *Eco*RI-*Sal*I fragment encoding IFNγ 4-146 from plasmid PIN5GIF54.

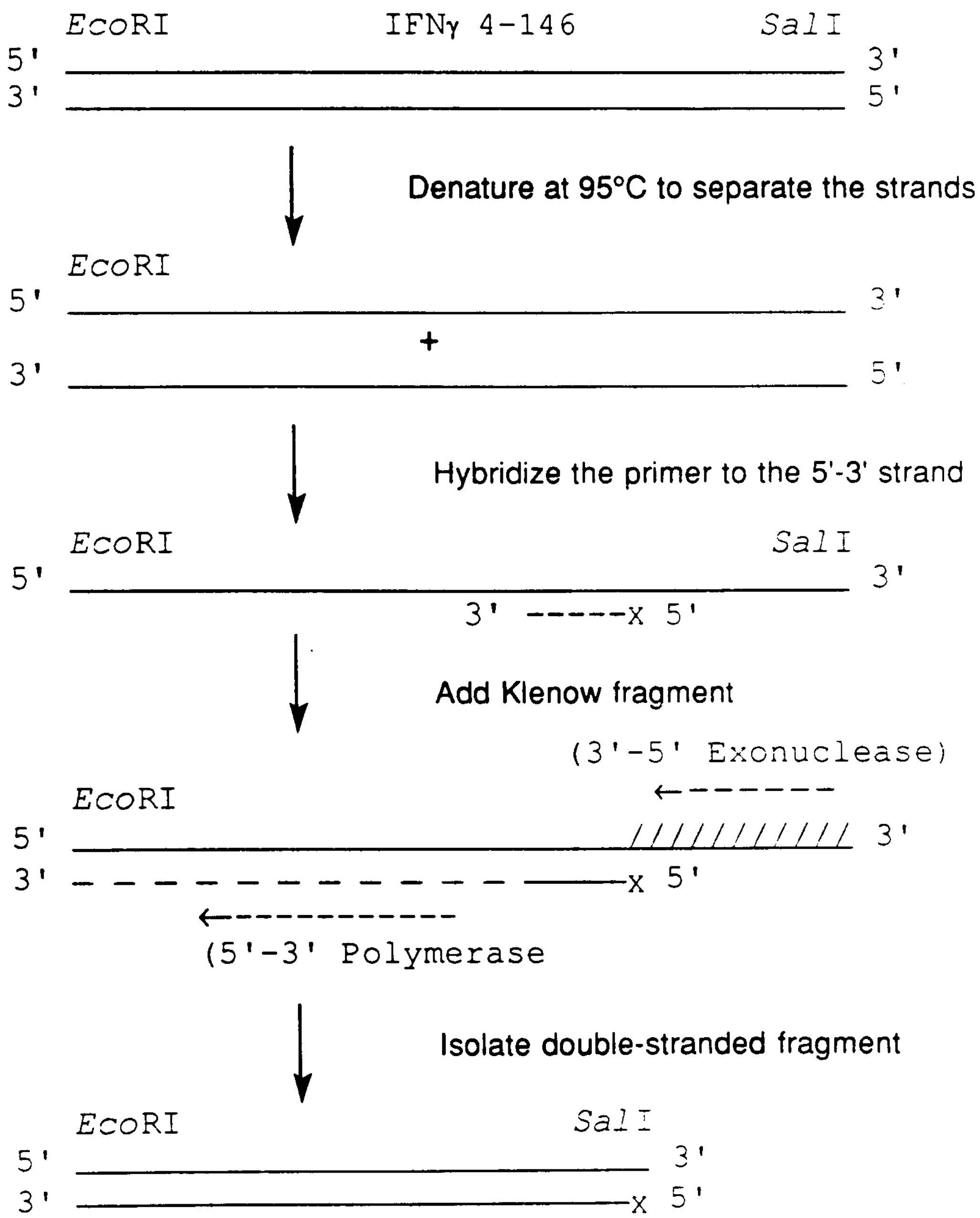


The resulting double-stranded fragment is then ligated into the desired plasmid, *e.g.* as shown in Figure 3.

Another method utilizes the technique of site-specific mutagenesis to introduce an appropriate nucleotide substitution for a desired amino acid substitution as described above or a stop triplet at the desired site, thus effectively terminating protein translation beyond this point. Alternatively, a gene sequence encoding the desired polypeptide sequence can be synthetically constructed.

The DNA needed to make the polypeptides of this invention can also be chemically synthesized, using the nucleotide sequence of IFNγ 4-134 as described above. Such chemical synthesis could be carried out using any of the known methods, *e.g.*, the phosphoramidite solid support method of Matteucci *et al.* [J. Am. Chem. Soc. 103:3185 (1981)].

The IFNγ 4-134 or its functional equivalents of this invention can also be reproduced by other methods of recombinant DNA methodology, including polymerase chain reaction [PCR] techniques, etc.

Once identified and isolated, the genes coding for IFNγ 4-134 or a functional equivalent thereof can be inserted into an appropriate cloning and/or expression vehicle containing the elements necessary for cloning, transcription and translation of the inserted gene sequences. Useful cloning vehicles may consist of segments of chromosomal, nonchromosomal and synthetic DNA sequences such as various known bacterial plasmids, phage DNA, combinations of plasmids and phage DNA such as plasmids that have been modified to employ phage DNA or other expression control sequences, or yeast plasmids. Any convenient one that is available may be used. Specific cloning vehicles which could be used include promoters such as *lpp*, *trp*, *lac*, *tac* or λ*pl*. A preferred promoter for expression is the *lpp* promoter. Additionally, the polypeptides of the invention can also be expressed in the periplasmic space by fusing the coding sequence to a signal peptide derived from *E. coli* proteins such as β-lactamase (Kadonaga *et al.*, "The Role of the β-lactamase Signal Sequence in the Secretion of Proteins by E. Coli", J. Biol. Chem., 259:2149-2154 (1984)), Phosphatase A (Oka *et al.*, "Efficacies of different Secretion Vectors for Secretion of Human Epidermal Growth Factor by E. Coli", Agric. Biol. Chem., 4:1099-1104 (1987)), or ompA (Skerra and Plückthun, "Assembly of a functional immunoglobulin Fv fragment in E. Coli", Science, 240:1038-1041)), etc.

The gene coding for IFNγ 4-134 or a functional equivalent thereof may be inserted into a cloning and/or expression vector by methods conventional in the art. For example, when the DNA sequences coding for IFNγ 4-134 or a functional equivalent thereof and for the desired cloning vehicle have been cut with the same restriction enzyme or enzymes, complementary DNA termini are produced which can be ligated. Alternatively, the cut ends that are produced may be modified by digesting single-stranded IFNγ 4-134 or vehicle DNA to produce blunt ends, or by filling in the single-stranded termini with an appropriate DNA polymerase. In this way, blunt-end ligation with an enzyme such as T4 DNA ligase may be employed to ligate the appropriate DNA coding for IFNγ 4-134 or a functional equivalent thereof into an appropriate cloning or expression vehicle. Also, linkers may be ligated onto the DNA termini. Such linkers may comprise specific oligonucleotide sequences that encode restriction site recognition sequences. The cleaved vector and the gene coding for IFNγ 4-134 or a functional equivalent thereof may also be modified by homopolymeric tailing, as described by Morrow [Methods in Enzymology, **68**:3 (1979)].

Many of the cloning and/or expression vehicles that may be used in this invention contain one or more marker activities that may be used to select for desired transformants, such as ampicillin and tetracycline resistance in pBR322, ampicillin resistance and β-galactosidase activity in pUC8, and ampicillin resistance in pEV-vrf2. Selection of host cells into which such vectors have been inserted is greatly simplified when the host cells otherwise lack the activities contributed by the vectors.

The determination of an appropriate host organism is affected by a number of factors, as is known in the art. These factors include, for example, compatibility with the chosen vector, toxicity of proteins encoded by the hybrid plasmid, ease of recovery of the desired protein, expression characteristics, biosafety and costs. Suitable hosts for use in the present invention include unicellular organisms such as plant cell lines, mammalian cell lines (such as COS7, NS-1 or CHO cell lines), yeast cells, insect expression systems (such as *Bombyx mori* or *Spodoptera frugiperda*) and bacteria (*e.g.*, *E. coli*).

Transfer of the recombinant cloning and/or expression vector into the host cell may be accomplished by methods conventional in the art. Depending upon the particular vector/host cell system, such transfer may be effected by transformation, transduction or transfection. Once such a modified host cell is produced, the cell can be cultured and the protein expression product may be isolated from the culture in a conventional manner.

## MATERIALS AND METHODS

### PLASMIDS, BACTERIA, AND ENZYMES

The Hu IFNγ gene used in this working example was chemically synthesized by the method of Tanaka *et al.*, Nucl. Acids Res. **11**, 1707-1723 (1983). This gene codes for the 146 amino acids of the mature Hu IFNγ gene (Gray *et al.*, Nature, cited above) and includes a methionine initiation codon. The 5'- and 3'-ends of the gene are flanked by *Eco*RI and *Sal*I sites, respectively. The initial three codons (coding for Cys-Tyr-Cys) were removed by primer repair by the method described by Gray *et al.*, (Proc. Natl. Acad. Sci. USA, cited above). Thus, all the constructions in this working example have the sequence Met-Gln-Asp-Pro ... at the amino terminal, where Gln is amino acid No. 4 of the full sequence (1-146). This gene having a Met at the amino terminal and the amino acids 4-146 of the full length wild-type IFNγ was used to prepare the IFNγ 4-134 and IFNγ 4-137, 4-135, 4-132 and 4-125 deletion mutants by either the primer repair method or the site-specific mutagenesis described below. The expression plasmid used was similar to pGIF5 disclosed by Tanaka *et al.* (cited above), except that the *lpp* promoter was substituted for the *lac* UV5 promotor. *E. coli* 294 was used for all expression work and plasmid isolation work in this example. All necessary enzymes were purchased from New England Biolabs.

1. CONSTRUCTION OF PIN5TGIF54 AND PIN5T4

1.A. CONSTRUCTION OF PIN5GIF54

PGIF54 and PIN1A2 were used as the starting plasmids for the construction of PIN5GIF54 (Fig. 1). An *Aat*I-*Eco*RI fragment from PGIF54 carrying the full-length IFNγ (1-146) gene was ligated into an *Aat*I-*Eco*RI-cut PIN1A2 plasmid. The final plasmid PIN5GIF54 contains the full-length IFNγ gene under the control of the *lpp* promoter.

PGIF54 is also known as SBMG105; it is available from Fermentation Research Institute, the agency of Industrial Science and Technology, 1-1-3, Higashi Tukuba Ibaragi, Japan, and is described in EPA 0 134 673. PIN1A2 (Construction of versatile expression cloning vehicles using the lipoprotein gene of *E. coli*; K. Nakamura and M. Inouye, EMBO J., Vol. 1 No. 6, 771-775, 1982) was used as the source of the *lpp* promoter.

1.B. CONSTRUCTION OF PIN5T4

The ampicillin-resistance gene in PIN5TGIF54 was replaced by the tetracycline-resistance gene in the following manner: An *Eco*RI-*Ava*I fragment that was converted into a blunt-ended fragment using Klenow was derived from the plasmid pBR322 and ligated into PIN5GIF54 that had been cut with *Sca*I. This step introduced the tetracycline-resistance gene as a selection marker and inactivated the ampicillin-resistance gene by insertional deletion. An *Eco*RI-*Sal*I synthetic fragment carrying the IFNγ (4-146) gene (Tanaka *et al.*, "Expression in *E. coli* of chemically synthesized gene for the Hu IFNγ"; Nucl. Acids. Res., 11:6, 1707-1723 (1983)) was used to replace the IFNγ (1-146) gene in the plasmid PIN5GIF54. PIN5GIF54 was digested with *Eco*RI and *Sal*I, and the synthetic fragment carrying the IFNγ (4-146) gene was inserted by ligation to give the plasmid PIN5T4 (see Fig. 2).

2. DERIVATION OF PGIF4-125 AND PGIF4-137

The *Eco*RI-*Sal*I fragment containing the coding sequence for full length gamma interferon was isolated from PIN5GIF54 and subjected to primer repair by the method of Gray *et al.* (Proc. Natl. Acad. Sci. USA, cited above). Synthetic oligomer primers to the carboxy-terminal sequence of IFNγ (given in SEQ IN NOS: 2 and 3, and showing use (in Scheme I) of the primer repair method of Gray *et al.* to provide a fragment encoding the desired fraction of IFNγ) were prepared by means of the Applied Biosystems DNA Synthesiser and were designed so as to introduce stop codons to give the analogs IFNγ 4-125 and 4-137. The design of these primers also included introduction of a *Sal*I site at the end of the coding region.

The primer-repaired fragments were ligated with the plasmid PIN5GIF54 that had been cut with *Sal*I, digested with Klenow to produce blunt ends, and digested with *Eco*RI. The resulting plasmids were named pGIF1-125 and pGIF1-137. In order to obtain pGIF4-125 and pGIF4-137, the *Hind*III-*Sal*I fragments from these plasmids were cut out and ligated into an *Hind*III-*Sal*I cut PIN5T4 plasmid. The resulting plasmids were named pGIF4-125 and pGIF4-137 (see Figure 3).

3. DERIVATION OF PGIF4-132, PGIF4-134 AND PGIF4-135

All of these plasmids were obtained by the site-directed mutagenesis method described by Kunkel (Proc. Natl. Acad. Sci. USA, 1985, 82:488-492). In order to do this the F1 origin of replication was introduced into pGIF4-137. The first step included introduction of a synthetic double-stranded oligomeric fragment containing the *Sac*I and *Bam*HI sites into the *Ava*I site of pGIF4-137 to give the plasmid pJF13-3 (see Figure 4). The F1 origin of replication was derived from pTZ19U (Mead *et al.*, Prot. Eng. **1**, 67-74 (1986); and BIO-RAD LABS. -- Muta-Gene phage and *in vitro* mutagenesis kit, catalog no. 170-3576) on a *Sac*I-*Bam*HI fragment which was ligated with a *Sac*I-*Bam*HI-cut pJF13-3 plasmid giving the plasmid pJF13-3-6 containing the IFNγ4-137 gene. This plasmid was used for all further manipulations for deriving pGIF4-132.

3.A. Mutagenic Primers

The primer of SEQ ID NO: 4 was used. This oligomer was doped at each position with the other three deoxynucleotide triphosphates. (During the synthesis of the oligomer, each deoxynucleotide triphosphate was mixed with a mixture of the other three deoxynucleotide triphosphates to about 5% of the total (v/v). pGIF4-132 was one of the mutants isolated from a library of several hundred mutants by virtue of a mutation that introduced a STOP codon (TAG) at position 133.)

The primers for pGIF4-134 and pGIF4-135 were as given in SEQ ID NOS: 5 and 6 respectively. These oligomeric primers can be used for primer repair and thus for defining the carboxy-terminal deletion mutant; they introduce a STOP codon after position 134 or 135 respectively of Hu IFNγ. The adjacent base pairs A G at positions 12 and 13 in the primer of SEQ ID NO: 5 and CT at positions 11 and 12 in the primer of SEQ ID NO: 6 are mutations to introduce the necessary STOP codon.

All synthetic primers were made using an Applied Biosystems Synthesiser.

3.B. Preparation of single-stranded DNA

The plasmid pJF13-3-6 was transformed into *E. coli* CJ236 (dutung-; Joyce and Grindley, J. Bacteriol., 158:636 (1984)). Any other equivalent strain that is available could have been used. Amp-resistant and chloramphenicol-resistant colonies were isolated. pJF13-3-6/CJ236 was grown and infected with the helper phage M13KO7 as described by Vieira and Messing in Methods in Enzymology, **153**, 3-11, 1987; eds., R. Wu and L. Grossman. After overnight growth the cells were centrifuged and a one-fifth volume of 2.5M NaCl / 20% polyethylene glycol was added to the supernatant. The mixture was incubated on ice for 60 seconds and centrifuged. The pellet was resuspended in 3 ml of 10 mM Tris.HCl, pH 8, 1 mM EDTA solution. RNase (30 µg) was added and the mixture incubated at 37°C for 20 minutes, and then proteinase K (600 µg) was added and the incubation was continued at 65°C for 30 minutes. This solution was extracted four times with a phenol-chloroform (1:1) solution and once with chloroform, and sodium acetate was then added to a final concentration of 0.3 M, pH 5.2. Two volumes of ethanol were then added. The single-stranded DNA was collected by centrifugation, washed with 70% ethanol, dried *in vacuo* and redissolved in 100 µl of 10 mM Tris.HCl, 1 mM EDTA, pH 8 solution.

3.C. Site-specific Mutagenesis

Single-stranded DNA from pJF13-3-6 (see Step 3.B. above) was used to carry out site-specific mutagenesis. In all experiments, 2 pmoles of the synthetic oligomer (for pGIF4-132, pGIF4-134 and pGIF4-135) and 1 µg of the single-stranded DNA were annealed in 10 µl of 20 mM Tris.HCl, 2 mM $MgCl_2$, 50 mM NaCl, pH 7.4. The volume was increased to 30 µl by addition of 3 µl of 10X synthesis buffer (100 mM Tris.HCl, 50 mM $MgCl_2$, 20 mM Dithiothreitol, pH 7.4), 3 µl ATP, 3 µl of 1.25 mM of each deoxynucleotide triphosphate (*i.e.*, adenosine, thymidine, guanosine, cytosine), 9 µl water, 1 µl of Klenow and 1 µl of T4 DNA ligase. The reaction was incubated at 15°C overnight. The double-stranded DNA (pGIF4-132, pGIF4-134 or pGIF4-135) was then transformed into *E. coli* 294. Colonies were screened for the production of a shortened gamma interferon molecule on SDS-PAGE and further sequenced (by the method of Sanger *et al.*, Proc. Natl. Acad. Sci. USA, **74** 5463-5467 (1977)) to confirm identity. Appropriate *Eco*RI-*Sal*I fragments were isolated and cloned back into *Eco*RI-*Sal*I-cut PIN5T4.

4. PURIFICATION OF DELETION MUTANTS OF HU IFNγ

The deletion mutants were expressed in *E. coli* 294 by overnight growth at 37°C to $O.D._{660}$≈6. The cells were harvested, resuspended to $O.D._{660}$ = 120 in 20 mM Tris.HCl / 5 mM benzamidine, pH 7.5 (buffer A), and broken in a Manton-Gaulin homogenizer. Insoluble material was removed by centrifugation at 16000 x g for ten minutes at 4°C. The supernatant was dialysed overnight against ten volumes of 20 mM Tris.HCl, 1 mM NaCl, 5 mM benzamidine, pH 7.5 (buffer B), at 4°C. (All subsequent steps were performed at 4°C.) It was centrifuged at 16000 x g for ten minutes to remove insoluble material and was then loaded onto a 50 ml column of Chelating Sepharose 6B (Pharmacia) that had been charged with $NiSO_4$ in buffer B according to the manufacturers' instructions. The column was washed with buffer B until the $A_{280}$≦1 and was then eluted with buffer B containing 25 mM imidazole, pH 7.5. The eluted protein peak was dialysed against buffer A until the [NaCl]≦10 mM. The dialysed solution was freed of insoluble material as described above and was then charged onto a 20 ml column of SP-Sephadex (Pharmacia) equilibrated in buffer A. The column was eluted with a 200 ml gradient from 0.0 to 0.3 M NaCl in buffer A. The single major protein peak, containing the IFNγ deletion mutant more than 99% pure, was pooled. The specific antiviral activity of IFNγ 4-137 purified by this procedure was 2 x $10^8$ units/mg.

The activities of the IFNγ 4-134 and functional equivalents thereof of the invention and of the other deletion mutants as described above can be demonstrated by the methods A-C below:

A. CYTOFLUOROMETRIC DETERMINATION OF CLASS II MHC (HLA-DR)

U937 (ATCC Catalog No. CRL 1593), a human histiocytic cell-line, was cultured in RPMI 1640 containing 10% heat-inactivated fetal bovine serum (FBS), 1 mM sodium pyruvate, non-essential amino acids, and penicillin-streptomycin at 37°C and 5% $CO_2$ in a humidity-controlled incubator. Cells at 1 x $10^5$ and more than 98% viability were incubated for 48 hours in media supplemented with concentrations ranging from 0.1 to 10 ng per ml of the various IFNγ derivatives. On termination of the experiment, the cells were washed once with phosphate-buffered saline (PBS) containing 1% FBS, and then 20 μg of mouse $IgG_1$ anti-human HLA-DR (Atlantic Antibodies Inc.) in RPMI 1640 was added to the washed pellet. After incubation on ice for 30 minutes, the cells were washed with PBS containing 1% FBS and 50 μg of FITC goat anti-mouse IgG in RPMI 1640 and held on ice for 30 minutes. The cells were then washed twice in PBS containing 0.01% sodium azide, suspended in 1 ml of PBS, and analyzed using a Becton-Dickinson FACScan cytofluorometer. Controls for isotype, FITC mouse $IgG_1$, and second antibody were used for every sample. Little $F_c$-mediated binding or second antibody binding was observed. Photomultiplier threshold settings were made by using the isotype controls (channel 2 mean fluorescence intensity, MFI). At these settings, second antibody control was gaussian at channel 9 MFI. Shifts as high as channel 90 MFI were observed with incubation of high levels of IFNγ using HLA-DR directed antibodies. The results with IFNγ 4-137, IFNγ 4-135, IFNγ 4-134, IFNγ 4-132 and IFNγ 4-125 are shown in Column 3 of Table I below.

B. ANTIVIRAL ASSAYS

Cytopathic effect-inhibition assay was used for measuring antiviral activity on human foreskin cells (FS71), as described by Rubinstein *et al.*, J. Virol., 37:744-758 (1981). Other suitable cell-lines that can be used in this assay are MDBK cell line (ATCC No. 6071), human fibroblast cell lines GM2504A and AG1732 (Human genetic Mutant Repository, Camden N.J.) and the human cell line WISH (ATCC NO. CCL 25). Soluble extract from sonicated IFNγ-expressing cells was routinely assayed. Expression levels of the mutants were normalized to Hu IFNγ (4-137) control extract by scanning gels stained with Coomassie brilliant blue dye. Based on many replicate assay samples (all samples were assayed at least six times), antiviral assays were accurate to ±25-50%. The results with IFNγ 4-137, IFNγ 4-135, IFNγ 4-134, IFNγ 4-132 and IFNγ 4-125 are shown in Column 2 of Table I below.

C. RECEPTOR BINDING ASSAY

C.i. PREPARATION OF $^{125}$I-LABELED HU IFNγ

IFNγ at 5 μg/50 μl in 20 mM sodium phosphate buffer, pH 7.2, was reacted with 2 mCi of Bolton-Hunter reagent (Amersham, 2000 Ci/mmole) for 1-2 hours at room temperature and then quenched with 50 μl of 1 M glycine, pH 8. The reaction was separated on a G-25 column (9 ml total volume) and then on a 1 x 24 cm G-75 column, both in 20 mM sodium phosphate buffer, 150 mM NaCl, 0.2% gelatin, pH 7.2. The labeled peak from the second column eluting as the IFNγ dimer (30 kD) was pooled for assays.

C.ii. BINDING ASSAY

U937 cells, grown as above to about $10^6$ cells/ml, were harvested and resuspended to 1.25 x $10^7$ cells/ml in a cold RPMI 1640 medium and 10% heat-inactivated FBS containing 0.02% sodium azide.

FS71 cells, a primary human foreskin fibroblast cell line, were cultured in Eagle's Minimal Essential Medium containing 10% (v/v) heat-inactivated fetal bovine serum, 0.3% sodium bicarbonate, 20 mM Hepes (pH 7.5), 4 mM L-glutamine and penicillin-streptomycin at 37°C and 5% $CO_2$ in a humidity-controlled incubator. Immediately prior to assay, the cell monolayer was washed with phosphate-buffered saline and then incubated in a minimum volume of 0.25% trypsin at 37°C for 10 min. Cell sheets were disrupted by aspiration, washed, filtered through cheese-cloth to remove large cell aggregates, and resuspended to 1.25 x $10^7$ cells/ml in RPMI 1640 containing 10% (v/v) heat-inactivated fetal bovine serum and 0.02% sodium azide.

To each assay point was added 100 μl of U937 cells or FS71 cells, 50 μl of $^{125}$I-Hu IFNγ (4-137, 4-135, 4-134, 4-132, or 4-125) (50,000-100,000 dpms, 1ng) in a microtiter dish well. Ten or twenty assay points were run for each protein analyzed. After incubation at 4°C for 2 hours with shaking, the reaction mixture was spun through 150 μl of dioctyl phthalate:dibutyl phthalate (1:1, Eastman Kodak Company). Reaction mixtures containing FS71 cells were spun through 150 μl RPMI 1640 containing 10% (v/v) heat-inactivated fetal bovine serum, 0.02% sodium azide, and 5% (w/v) sucrose. The assay tubes were frozen in liquid nitrogen, and the cell pellets were excised and their radioactivity determined in a γ-counter. The results are shown in Columns 4 and 5 of Table I below.

Similar tests carried out on HeLa cells gave the following results in the activity tests in columns 2 and 3 respectively of Table I: 3% and 2-4%. Tumors or corresponding cell lines that are susceptible to treatment with IFNγ 4-134 can be identified by these test procedures, if necessary when carried out *in vitro* on a cell line derived from the tumor. By such procedures, tumors that are susceptible in this way to treatment with IFNγ 4-134 (in that it will increase their sensitivity to cytotoxic cells) can be identified.

## TABLE I. CARBOXYL TERMINAL DELETIONS

```
125                           132      134 135       137
-Pro-Ala-Ala-Lys-Thr-Gly-Lys-Arg-Lys-Arg-Ser-Gln-Met
```

| Hu IFNγ Deletion Mutant | Antiviral Activity on FS71 Fibroblasts[1] | HLA-DR Activity on U937 cells[2] | Receptor Binding Activity on U937 cells | Receptor Binding Activity on FS71 cells |
|---|---|---|---|---|
| 4-137* | 100% | 100% | 100% | 100% |
| 4-135 | 70 | 150 | 100 | 100 |
| 4-134 | 3 | 100 | 100 | 100 |
| 4-132 | 1 | 23 | 4 | 4 |
| 4-125 | 1 | 1 | 1 | 11 |

* All activities for this deletion mutant are defined as 100%; all the activities for the other deletion mutants are expressed as a percentage of the relevant activity of IFNγ 4-137.

1 On FS71 fibroblasts, IFNγ 4-137 has a specific activity of 2-3 x $10^8$ units/mg, and all antiviral activities of other IFNγs are expressed as a percentage of this activity.

2 4-137 gives 1/2 maximal response at 1-2 ng/ml.

Table I shows how deletions at the carboxyl terminus affect both the receptor binding and biological activity. The deletion-mutant IFNγ 4-134 of the present invention shows undiminished HLA-DR induction activity but has very low antiviral activity, and for this purpose is advantageous over deletion mutant IFNγ 4-132, which has similar low antiviral activity but significantly reduced HLA-DR induction activity.

These data also show that Hu IFNγ 4-134 has unique properties that make it a potentially useful inducer of HLA-DR induction without anti-viral effects and in particular without the side-effects associated with anti-viral activity. By virtue of these unique properties, Hu IFNγ 4-134 is likely to find therapeutic use in enhancing the immune response in immuno-suppressed patients and as an adjunct to chemotherapy in various kinds of human cancers.

Hu IFNγ 4-134 is also useful in increasing the antibody response in conjunction with a vaccine. The vaccines may for example be against bacterial or viral diseases and may be polyvalent; vaccines that can usefully incorporate IFNγ 4-134 or a functional equivalent thereof include those against diseases such as cholera, diphtheria, typhoid, paratyphoid, tetanus, pertussis, tuberculosis, poliomyelitis, smallpox, influenza, hepatitis, rabies, yellow fever, etc. It will of course be clear to those skilled in the art that no constituents of the vaccine should deactivate the IFNγ; under certain circumstances, it might be more appropriate to mix the IFNγ with the vaccine immediately before administration, especially when a veterinary vaccine is used.

The Hu IFNγ 4-134 or its functional equivalent of the invention can be administered as a pharmaceutical composition. Such compositions contain a therapeutically effective amount of Hu IFNγ 4-134 or a functional equivalent thereof and a pharmaceutical carrier or excipient. A pharmaceutical carrier can be any compatible, non-toxic substance suitable for delivering Hu IFNγ 4-134 or its functional equivalent to a patient. Sterile water, alcohol, fats, waxes, and inert solids may for example be included in a carrier, and pharmaceutically acceptable adjuvants (*e.g.*, buffering agents, dispersing agents) may also be incorporated. Generally, compositions useful for parenteral administration of such drugs are well known, *e.g.*, see Remington's Pharmaceutical Sciences, 14th Ed. (Mack Publishing Company, Easton, PA, 1980). Alternatively, compositions of the invention may be introduced into a patient's body by an implantable drug delivery system; *e.g.* see Urquhart *et al.*, Ann. Rev. Pharmacol. Toxicol., vol. 24, pgs. 199-236 (1984).

The Hu IFNγ 4-134 or its functional equivalent is normally administered parenterally, preferably intravenously. It can also be administered slowly, either by a conventional IV administration set or from a subcutaneous depot.

When administered parenterally, Hu IFNγ 4-134 or its functional equivalent will normally be formulated with a pharmaceutically acceptable parenteral vehicle in a unit dosage form suitable for injection (*e.g.*, a solution, suspension or emulsion). Such vehicles are inherently non-toxic and non-therapeutic. Examples of such vehicles are normal saline, Ringer's solution, dextrose solution, and Hank's solution. Non-aqueous vehicles such as fixed oils and ethyl oleate may also be used. A preferred vehicle is 5% dextrose/saline. The vehicle may contain minor amounts of additives such as substances that enhance isotonicity and chemical stability, *e.g.*, buffers and preservatives. Hu IFNγ 4-134 or its functional equivalent is preferably formulated in purified form substantially free of aggregates, degradation products and contaminating proteins, at concentrations of about 5 to 500 µg/ml, preferably 20 to 250 µg/ml.

Selecting an administration regimen for Hu IFNγ 4-134 or its functional equivalent depends on several factors, such as the polypeptide's serum turnover rate and the accessibility of the target cells to the polypeptide.

Determination of the proper dosage of a compound of the invention for a particular situation is within the skill of the art. Usually, treatment is initiated with dosages that are less than the optimum dose. Thereafter, the dosage is increased by small amounts until the optimum effect under the circumstances is reached. For convenience, the total daily dosage may be divided and administered in portions during the day if desired.

The amount and frequency of administration of Hu IFNγ 4-134 or its functional equivalent will be regulated according to the judgment of the attending clinician considering such factors as age, condition and size of the patient as well as severity of the symptoms being treated.

According to the administration regimen, the amount of Hu IFNγ 4-134 or its functional equivalent delivered to the patient is preferably maximized consistent with an acceptable level of side effects; and the amount delivered therefore depends in part on the severity of the disease being treated. Preferably, the dose is in the range of about 0.1 to 500 µg/kg per day, more preferably about 1 to 50 µg/kg per day.

A typical recommended dosage regimen is parenteral administration of from 1 µg/day to 5 mg/day, preferably 20 µg/day to 1 mg/day, in two to four divided doses to achieve therapeutic relief.

The descriptions of the foregoing embodiments of the invention have been presented for purpose of illustration and description. They are not intended to be exhaustive or to limit the invention to the precise forms disclosed, and obviously many modifications and variations are possible in the light of the above teachings. The embodiments were chosen and described to explain the principles of the invention and its practical application, so that others skilled in the art would thereby be enabled to use and practise appropriately such embodiments and modifications of the invention as are suited to the particular use contemplated. It is intended that the scope of the invention be defined by the claims appended hereto.

SEQUENCE LISTING

(1) GENERAL INFORMATION:

(i) APPLICANT: Narula, Satwant K.; Lundell, Daniel J.

(ii) TITLE OF INVENTION: Human interferon-γ, Functional Equivalents thereof and Methods of Using and Compositions Employing these Materials

(iii) NUMBER OF SEQUENCES: 6

(iv) CORRESPONDENCE ADDRESS:

(A) ADDRESSEE: Schering-Plough Corporation

(B) STREET: One Giralda Farms

(C) CITY: Madison

(D) STATE: New Jersey

(E) COUNTRY: USA

(F) ZIP: 07940

(v) COMPUTER READABLE FORM:

(A) MEDIUM TYPE: Floppy Disc

(B) COMPUTER: Apple Macintosh

(C) OPERATING SYSTEM: Macintosh 6.0.5

(D) SOFTWARE: Microsoft Word 4.00B

(vi) CURRENT APPLICATION DATA:

(A) APPLICATION NUMBER:

(B) FILING DATE:

(C) CLASSIFICATION:

(vii) PRIOR APPLICATION DATA:

(A) APPLICATION NUMBER: 576,245

(B) FILING DATE: 31st August 1990

(viii) ATTORNEY/AGENT INFORMATION:

(A) NAME: Blasdale, John H. C.

(B) REGISTRATION NUMBER: 31,895

(C) REFERENCE/DOCKET NUMBER: JB0159K

(ix) TELECOMMUNICATION INFORMATION:

(A) TELEPHONE: 201-822-7398

(B) TELEFAX: 201-822-7039

(C) TELEX: 219165

(2) INFORMATION FOR SEO ID NO: 1:

(i) SEQUENCE CHARACTERISTICS:

(A) LENGTH: 146 amino acids

(B) TYPE: amino acid

(C) STRANDEDNESS: single

(D) TOPOLOGY: linear

(vi) ORIGINAL SOURCE:

(A) ORGANISM: human

(x) PUBLICATION INFORMATION:

(A) AUTHORS: Gray *et al.*

(B) TITLE: Expression of human immune interferon cDNA in *E. coli* and monkey cells

(C) JOURNAL: *Nature*

(D) VOLUME: **295**

(E) ISSUE: 11 February 1982

(F) PAGES: 503-508

(G) DATE: 1982

(xi) SEQUENCE DESCRIPTION: SEQ ID NO: 1:

```
Cys Tyr Cys Gln Asp Pro Tyr Val Lys Glu Ala Glu Asn Leu Lys
                5                   10                  15
Lys Tyr Phe Asn Ala Gly His Ser Asp Val Ala Asp Asn Gly Thr
                20                  25                  30
Leu Phe Leu Gly Ile Leu Lys Asn Trp Lys Glu Glu Ser Asp Arg
                35                  40                  45
Lys Ile Met Gln Ser Gln Ile Val Ser Phe Tyr Phe Lys Leu Phe
                50                  55                  60
Lys Asn Phe Lys Asp Asp Gln Ser Ile Gln Lys Ser Val Glu Thr
                65                  70                  75
Ile Lys Glu Asp Met Asn Val Lys Phe Phe Asn Ser Asn Lys Lys
                80                  85                  90
Lys Arg Asp Asp Phe Glu Lys Leu Thr Asn Tyr Ser Val Thr Asp
                95                  100                 105
Leu Asn Val Gln Arg Lys Ala Ile His Glu Leu Ile Gln Val Met
                110                 115                 120
Ala Glu Leu Ser Pro Ala Ala Lys Thr Gly Lys Arg Lys Arg Ser
                125                 130                 135
Gln Met Leu Phe Arg Gly Arg Arg Ala Ser Gln
                140                 145
```

(3) INFORMATION FOR SEQ ID NO: 2:

(i) SEQUENCE CHARACTERISTICS:

(A) LENGTH: 24 bases

(B) TYPE: nucleic acid

(C) STRANDEDNESS: single

(D) TOPOLOGY: linear

(ii) MOLECULE TYPE: DNA

(vii) IMMEDIATE SOURCE: [Synthetic]

(ix) FEATURE:

(D) OTHER INFORMATION: Encodes amino acid residues 119-125 of Hu IFNγ. This oligomer can be used for primer repair and thus for defining the carboxy-terminal deletion mutant; it introduces a STOP codon after position 125 of Hu IFNγ. The initial C reconstructs a filled-in *Sal*I end to the full *Sal*I recognition sequence (5'-GTCGAC-3'). The first codon CTA represents the STOP codon (corresponding to TAG on the coding strand). The next codon, CGG, encodes the last amino acid for the polypeptide, *viz* 125 Pro.

(xi) SEQUENCE DESCRIPTION: SEQ ID NO: 2:

CTA CGG GGA CAG TTC AGC CAT AAC 24

(3) INFORMATION FOR SEQ ID NO: 3:

(i) SEQUENCE CHARACTERISTICS:

(A) LENGTH: 24 bases

(B) TYPE: nucleic acid

(C) STRANDEDNESS: single

(D) TOPOLOGY: linear

(ii) MOLECULE TYPE: DNA

(vii) IMMEDIATE SOURCE: [Synthetic]

(ix) FEATURE:

(D) OTHER INFORMATION: Encodes amino acid residues 131-137 of Hu IFNγ. This oligomer can be used for primer repair and thus for defining the carboxy-terminal deletion mutant; it introduces a STOP codon after position 137 of Hu IFNγ. The initial C reconstructs a filled-in *Sal*I end to the full *Sal*I recognition sequence (5'-GTCGAC-3'). The first codon CTA represents the STOP codon (corresponding to TAG on the coding strand). The next codon, CGG, encodes the last amino acid for the polypeptide, *viz* 137 Met.

(xi) SEQUENCE DESCRIPTION: SEQ ID NO: 3:

CTA CAT CTG AGA TCT TTT TAC GCT 24

(3) INFORMATION FOR SEO ID NO: 4:

(i) SEQUENCE CHARACTERISTICS:

(A) LENGTH: 52 bases

(B) TYPE: nucleic acid

(C) STRANDEDNESS: single

(D) TOPOLOGY: linear

(ii) MOLECULE TYPE: DNA

(vii) IMMEDIATE SOURCE: [Synthetic]

(ix) FEATURE:

(D) OTHER INFORMATION: Synthetic mutagenic primer: during its preparation, it was doped at each position with the other three deoxynucleotide triphosphates; used to isolate mutants of Hu IFNγ.

(xi) SEQUENCE DESCRIPTION: SEQ ID NO: 4:

AAC TGG AAA GAA GAA TCT GAC CGT AAA AAT CAT GCA GTC TCA 42
GAT CGT TTC 52

(3) INFORMATION FOR SEQ ID NO: 5:

(i) SEOUENCE CHARACTERISTICS:

(A) LENGTH: 22 bases

(B) TYPE: nucleic acid

(C) STRANDEDNESS: single

(D) TOPOLOGY: linear

(ii) MOLECULE TYPE: DNA

(vii) IMMEDIATE SOURCE: [Synthetic]

(ix) FEATURE:

(D) OTHER INFORMATION: Primer for pGIF4-134. This oligomer is used for primer repair and thus for defining the carboxy-terminal deletion mutant; it introduces a STOP codon after position 134 of Hu IFNγ. Adjacent base pairs A G at positions 12 and 13 are mutations to introduce the necessary STOP codon.

(xi) SEQUENCE DESCRIPTION: SEQ ID NO: 5:

GCG TAA AAG ATA GCA GAT GTA G 22

(3) INFORMATION FOR SEQ ID NO: 6:

(i) SEQUENCE CHARACTERISTICS:

(A) LENGTH: 22 bases

(B) TYPE: nucleic acid

(C) STRANDEDNESS: single

(D) TOPOLOGY: linear

(ii) MOLECULE TYPE: DNA

(vii) IMMEDIATE SOURCE: [Synthetic]

(ix) FEATURE:

(D) OTHER INFORMATION: Primer for pGIF4-135. This oligomer is used for primer repair and thus for defining the carboxy-terminal deletion mutant; it introduces a STOP codon after position 135 of Hu IFNγ. Adjacent base pairs CT at positions 11 and 12 are mutations to introduce the necessary STOP codon.

(xi) SEQUENCE DESCRIPTION: SEQ ID NO: 6:

GTA AAA GAT CCT AGA TGT AGT C 22

## Claims

1. The use of Hu $IFN_{\gamma}$ 4-134 polypeptide, whose sequence consists of residues numbered 4 to 134 of SEQ ID NO: 1 herein, or of said sequence preceded by Met, for the manufacture of a medicament useful for increasing the antibody response in conjunction with a vaccine.

2. The use of Hu $IFN_{\gamma}$ 4-134 polypeptide, whose sequence consists of residues numbered 4 to 134 of SEQ ID NO: 1 herein, or of said sequence preceded by Met, for the preparation of a medicament useful for increasing the susceptibility of tumor cells to cytotoxic cells.

3. The use of Hu $IFN_{\gamma}$ 4-134 polypeptide whose sequence consists of residues numbered 4 to 134 of SEQ ID NO: 1 herein, or of said sequence preceded by Met, for the manufacture of a medicament useful for increasing the expression of surface antigens by tumor cells.

4. Use as claimed in any of claims 1 to 3 wherein the sequence of the recombinant human interferon-γ consists of residues numbered 4 to 134 of SEQ ID NO: 1 herein.

## Patentansprüche

1. Verwendung eines Hu IFNγ 4-134-Polypeptids, dessen Sequenz aus den mit 4 bis 134 numerierten Resten der SEQ ID Nr. 1 hierin oder aus dieser Sequenz, der Met vorangeht, besteht, zur Herstellung eines Arzneimittels, das zusammen mit einem Impfstoff zum Erhöhen der Antikörperantwort brauchbar ist.

2. Verwendung eines Hu IFNγ 4-134-Polypeptids, dessen Sequenz aus den mit 4 bis 134 numerierten Resten der SEQ ID Nr. 1 hierin oder aus dieser Sequenz, der Met vorangeht, besteht, zur Herstellung eines Arzneimittels, das zur Erhöhung der Empfindlichkeit von Tumorzellen gegen zytotoxische Zellen brauchbar ist.

3. Verwendung eines Hu IFNγ 4-134-Polypeptids, dessen Sequenz aus den mit 4 bis 134 numerierten Resten der SEQ ID Nr. 1 hierin oder aus dieser Sequenz, der Met vorangeht, besteht, zur Herstellung eines Arzneimittels, das zur Erhöhung der Expression von Oberflächenantigenen durch Tumorzellen brauchbar ist.

4. Verwendung wie in einem der Ansprüche 1 bis 3 beansprucht, wobei die Sequenz des rekombinanten Human-Interferon-γ aus den mit 4 bis 134 numerierten Resten der SEQ ID Nr. 1 hierin besteht.

## Revendications

1. Utilisation du polypeptide Hu IFNγ 4-134 dont la séquence se compose de résidus numérotés 4 à 134 de SEQ ID NO : 1 ici, l'une desdites séquences étant précédée de Met, pour la fabrication d'un médicament utile pour augmenter la réponse des anticorps en association avec un vaccin.

2. Utilisation du polypeptide Hu IFNγ 4-134 dont la sépence se compose de résidus numérotés 4 à 134 de SEQ ID N0 : 1 ici, ou de ladite séquence précédée de Met, pour la préparation d'un médicament utile pour augmenter la sensibilité de cellules tumorales à des cellules cytotoxiques.

3. Utilisation du polypeptide Hu IFNγ 4-134 dont la séquence se compose de résidus numérotés 4 à 134 de SEQ ID NO : 1 ici, ou de ladite séquence précédée de Met, pour la fabrication d'un médicament utile pour augmenter l'expression des antigènes de surface par les cellules tumorales.

4. Utilisation selon l'une quelconque des revendications 1 à 3 où la séquence de l'interféron-γ humain recombinant se compose de résidus numérotés 4 à 134 de SEQ ID NO : 1 ici.

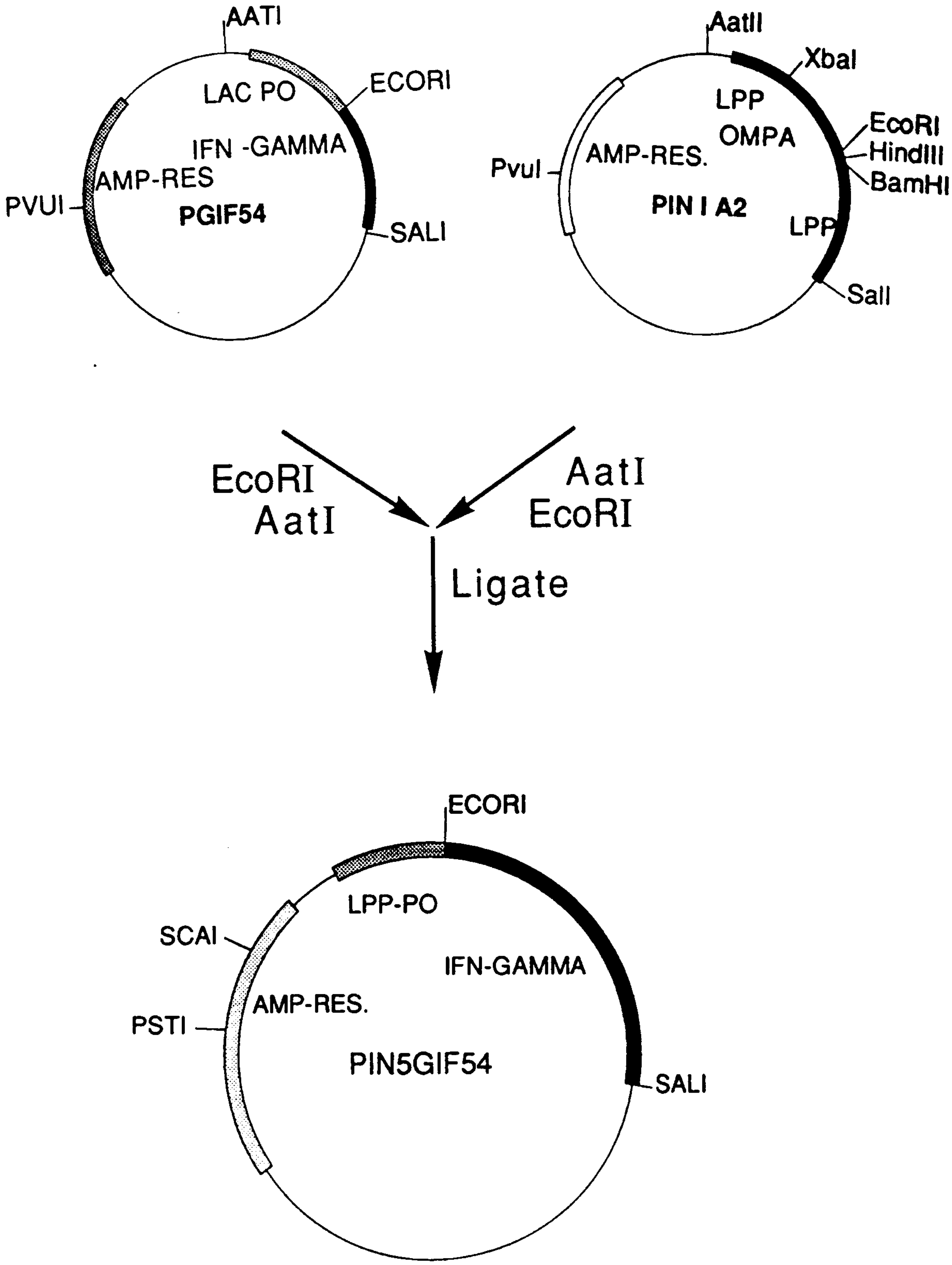
AATI
LAC PO
ECORI
IFN -GAMMA
AMP-RES
PVUI
PGIF54
SALI
AatII
XbaI
LPP
OMPA
EcoRI
HindIII
BamHI
PvuI
AMP-RES.
PIN I A2
LPP
SalI
EcoRI
AatI
AatI
EcoRI
Ligate
ECORI
LPP-PO
SCAI
IFN-GAMMA
AMP-RES.
PSTI
PIN5GIF54
SALI

*FIGURE 1*

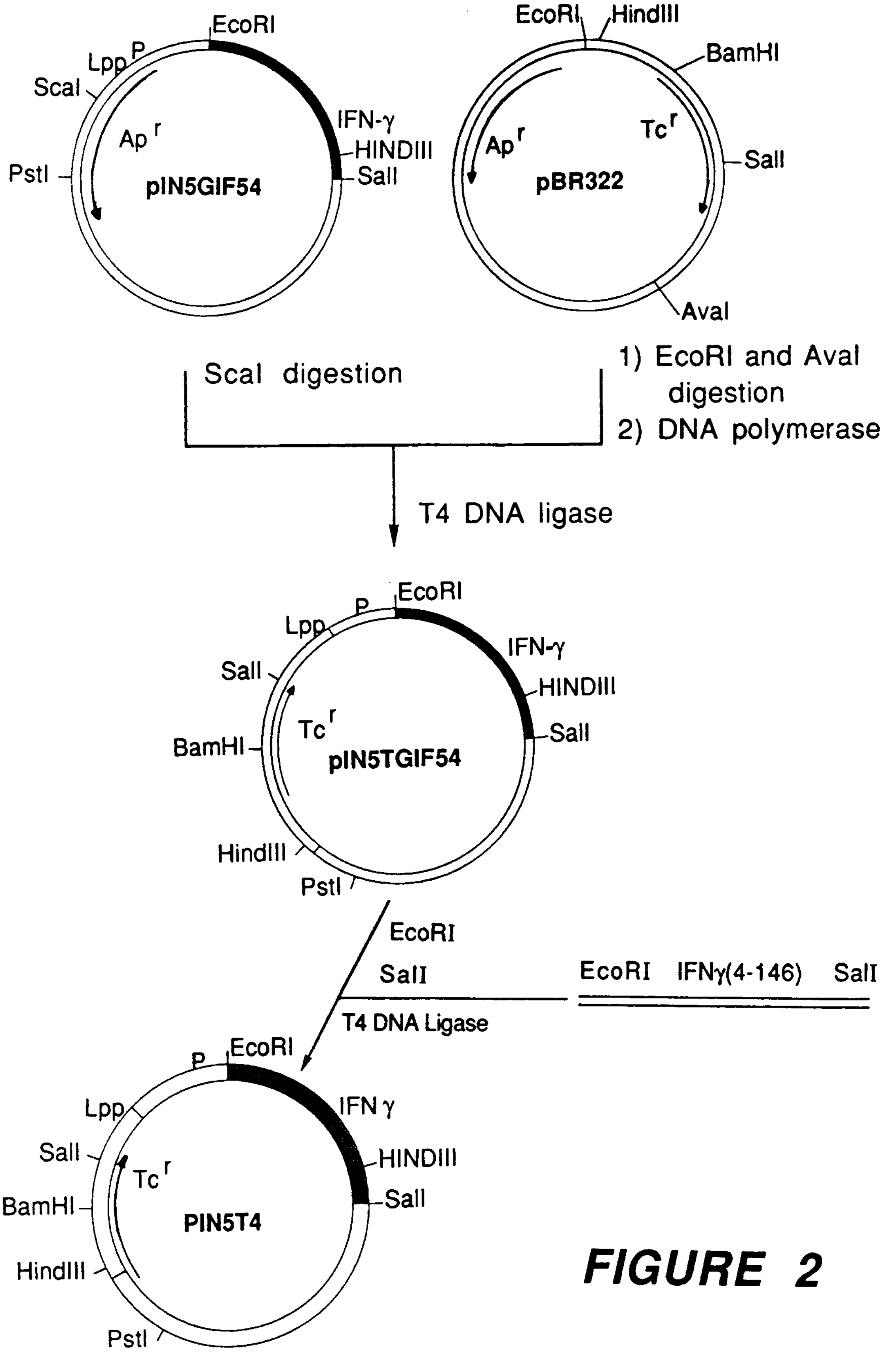
EcoRI
Lpp
P
ScaI
IFN-γ
Ap r
HINDIII
PstI
SalI
pIN5GIF54
EcoRI
HindIII
BamHI
Tc r
Ap r
SalI
pBR322
AvaI
ScaI digestion
1) EcoRI and AvaI digestion
2) DNA polymerase
T4 DNA ligase
EcoRI
P
Lpp
IFN-γ
SalI
HINDIII
Tc r
SalI
BamHI
pIN5TGIF54
HindIII
PstI
EcoRI
SalI
EcoRI IFNγ(4-146) SalI
T4 DNA Ligase
P
EcoRI
Lpp
IFN γ
SalI
HINDIII
Tc r
SalI
BamHI
PIN5T4
HindIII
PstI

*FIGURE 2*

# FIGURE 3

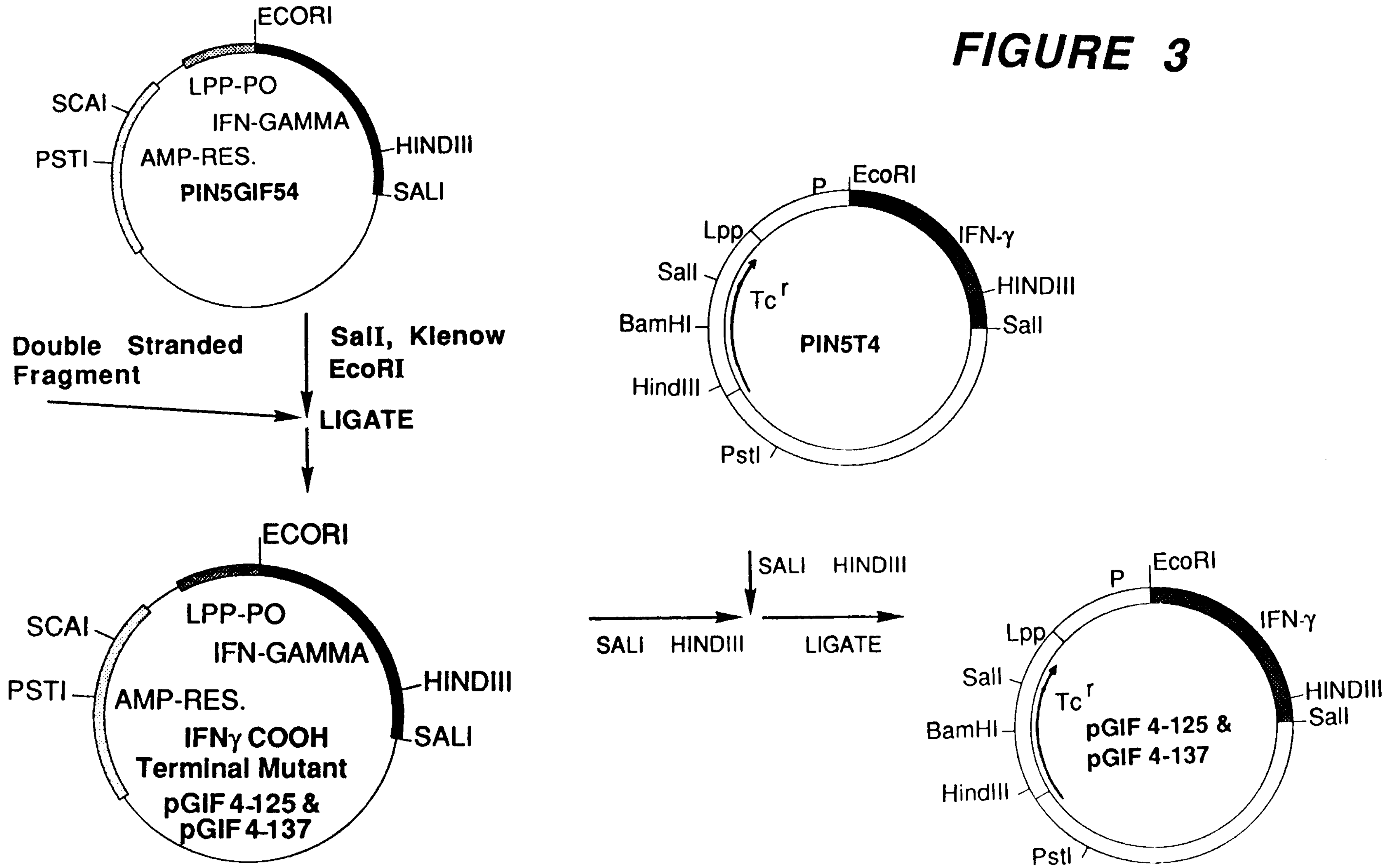
ECORI
SCAI
PSTI
LPP-PO
IFN-GAMMA
AMP-RES.
PIN5GIF54
HINDIII
SALI
Double Stranded Fragment
SalI, Klenow
EcoRI
LIGATE
ECORI
SCAI
PSTI
LPP-PO
IFN-GAMMA
AMP-RES.
IFNγ COOH
Terminal Mutant
pGIF 4-125 &
pGIF 4-137
HINDIII
SALI
P
EcoRI
Lpp
SalI
BamHI
HindIII
PstI
Tc r
PIN5T4
IFN-γ
HINDIII
SalI
SALI HINDIII
SALI HINDIII
LIGATE
P
EcoRI
Lpp
SalI
BamHI
HindIII
PstI
Tc r
pGIF 4-125 &
pGIF 4-137
IFN-γ
HINDIII
SalI

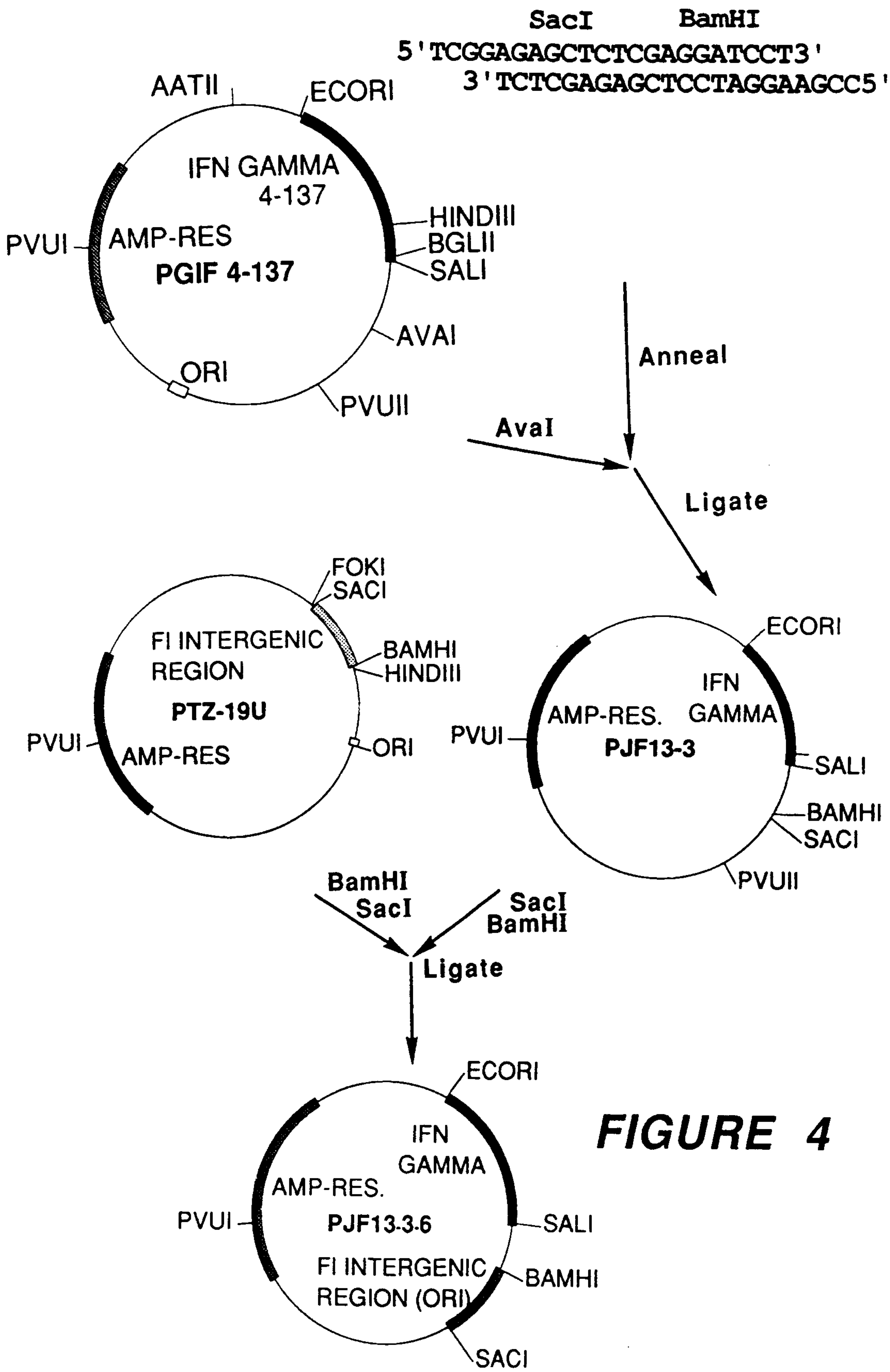
SacI
BamHI
5'TCGGAGAGCTCTCGAGGATCCT3'
3'TCTCGAGAGCTCCTAGGAAGCC5'
AATII
ECORI
IFN GAMMA
4-137
HINDIII
BGLII
SALI
PVUI
AMP-RES
PGIF 4-137
AVAI
ORI
PVUII
Anneal
AvaI
Ligate
FOKI
SACI
FI INTERGENIC
REGION
BAMHI
HINDIII
PTZ-19U
PVUI
AMP-RES
ORI
ECORI
IFN
GAMMA
AMP-RES.
PVUI
PJF13-3
SALI
BAMHI
SACI
PVUII
BamHI
SacI
SacI
BamHI
Ligate
ECORI
IFN
GAMMA
AMP-RES.
PVUI
PJF13-3-6
SALI
FI INTERGENIC
REGION (ORI)
BAMHI
SACI

*FIGURE 4*